(19) Europäisches Patentamt
European Patent Office
Office européen des brevets

(11) Veröffentlichungsnummer: **0 103 136**
**B1**

# (12) EUROPÄISCHE PATENTSCHRIFT

(45) Veröffentlichungstag der Patentschrift: **18.05.88**

(51) Int. Cl.⁴: **C 07 C 53/19**

(21) Anmeldenummer: **83107634.4**

(22) Anmeldetag: **03.08.83**

(54) **5,5-Dichlor-3,3-dimethylpentansäure und ein Verfahren zu ihrer Herstellung.**

(30) Priorität: **14.08.82 DE 3230274**

(43) Veröffentlichungstag der Anmeldung: **21.03.84 Patentblatt 84/12**

(45) Bekanntmachung des Hinweises auf die Patenterteilung: **18.05.88 Patentblatt 88/20**

(84) Benannte Vertragsstaaten: **CH DE FR GB LI**

(56) Entgegenhaltungen:
**US-A-4 235 780**

**CHEMICAL ABSTRACTS EIGHTH COLLECTIVE INDEX, Vol. 66-75, 1967-1971, COLUMBUS, OHIO, (US). Subjects: Valeric acid, Seite 32.636s, Spalte 3 & Vol. 68, Seite 5708, 1968, nr 59161k. N. SHAMP: "Syntheses with 2,2-di-chlorodimedone". & Verh. Kon. Vlaam. Acad. Wetensch., Belg., Kl. Wetensch. 28(90), 166 pp**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Arlt, Dieter, Prof. Dr., Rybniker Strasse 2, D-5000 Köln 80 (DE)**

EP 0 103 136 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft 5,5-Dichlor-3,3-dimethyl-pentansäure und ein Verfahren zu ihrer Herstellung.

Es ist bekannt geworden, 4,4-Dimethyl-3,4-dihydro-α-pyron nach dem im folgenden skizzierten Reaktionsweg zu erhalten (vgl. EP-OS 31 932):

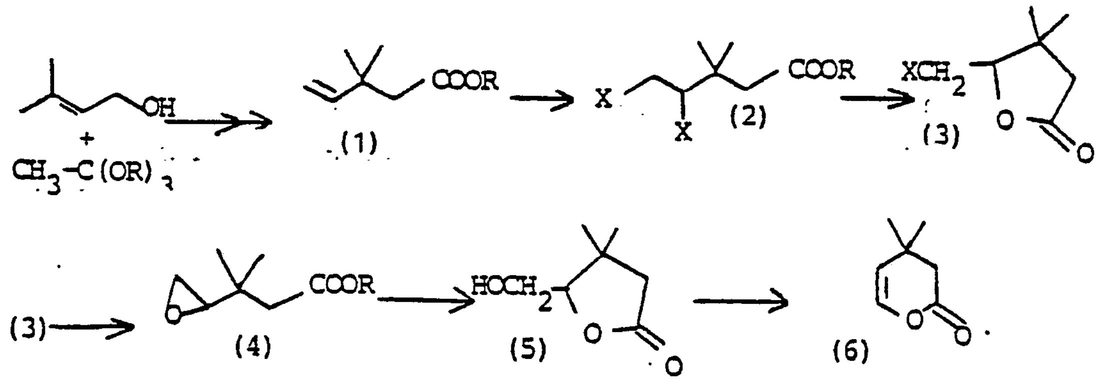

(1) (2) (3) (4) (5) (6)

Hierbei entsteht die Verbindung (2), welche mit der erfindungsgemäßen Verbindung 5,5-Dichlor-3,3-dimethyl-pentan-Säure strukturell vergleichbar ist. Wie aus dem obigen Reaktionsschema ersichtlich, sind ausgehend von der Verbindung der Formel (2) mindestens vier ziemlich komplizierte Stufen notwendig, um die Verbindung 4,4-Dimethyl-3,4-dihydro-α-pyron (der Formel (6)) zu erhalten.

Ausgehend von der erfindungsgemäßen Verbindung 5,5-Dichlor-3,3-dimethylpentansäure der Formel (I)

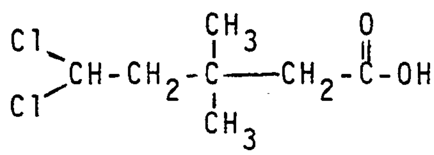 (I)

wird nun ein neuer und erfinderischer Weg zur bekannten Verbindung 4,4-Dimethyl-3,4-dihydro-α-pyron wie folgt eröffnet:

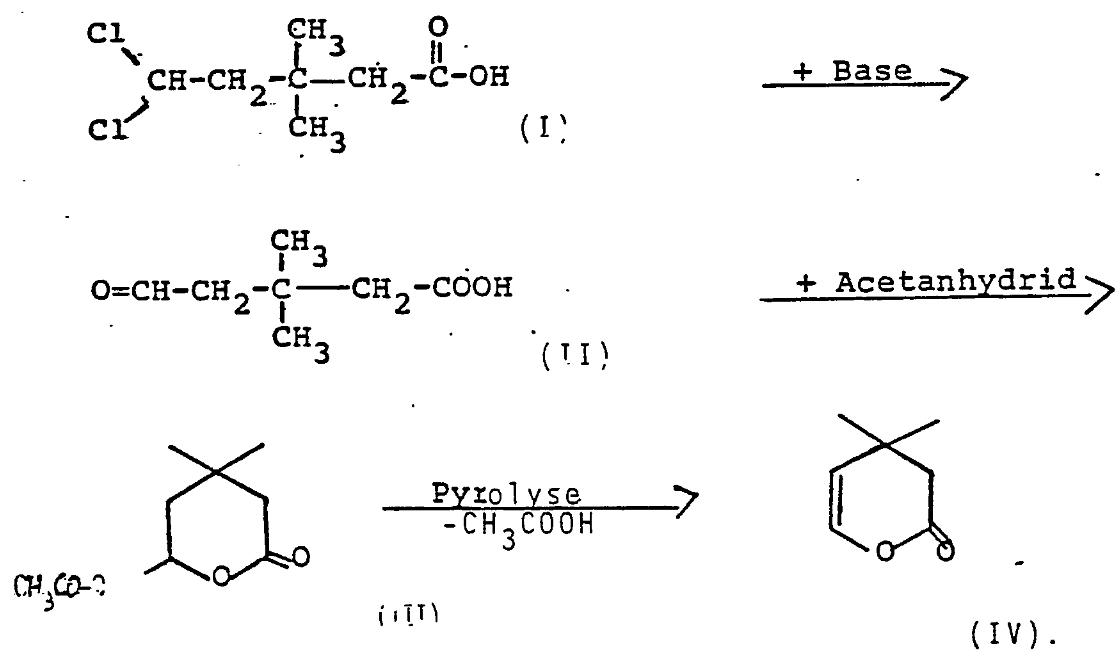


(I) (II) (III) (IV).

Der Verfahrensschritt I → (II) verläuft unerwartet, weil an Stelle der Hydrolyse zum Aldehyd eine Eliminierung hätte erwartet werden müssen. Weiterhin sind nur 3 Stufen zum Erhalt der Verbindung (IV) notwendig, oder sogar nur zwei Stufen, falls man, wie bevorzugt, das Valerolacton (III) nicht isoliert.

Die vorliegende Erfindung betrifft weiterhin ein Verfahren zur Herstellung von 5,5-Dichlor-3,3-dimethyl-pen-

tansäure, welches dadurch gekennzeichnet ist, daß man 1,1,5,5-Tetrachlor-3,3-dimethyl-pentan der Formel (V)

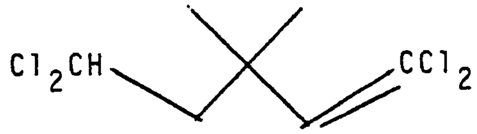

(V)

sauer hydrolysiert.

Die Hydrolyse wird in einem Temperaturbereich von ca. 20° - 100°C, vorzugsweise von 40° - 70°C, durchgeführt. Im allgemeinen wird ein Überschuß an Säure eingesetzt, mindestens jedoch soll das Reaktionsgemisch die dem eingesetzten Produkt der Formel V äquivalente Menge Wasser enthalten. Zur Hydrolyse sind neben Schwefelsäure auch andere starke Säuren wie z. B. Phosphorsäure, Methansulfonsäure, Trifluouressigsäure und Gemische von Ameisensäure und Salzsäure geeignet.

Will man die Säure der Formel I isolieren, so wird das Reaktionsgemisch mit Wasser verdünnt und die Säure (I) mit Lösungsmittel, wie z. B. Chlorkohlenwasserstoffen extrahiert. Bei Verwendung destillierbarer Säuregemische, wie z. B. der nach der Hydrolyse mit Trifluoressigsäure oder Ameisensäure-Salzsäure erhaltenen Reaktionsgemische, wird destillativ aufgearbeitet, um die Säure (I) rein zu gewinnen.

1,1,5,5-Tetrachlor-3,3-dimethyl-penten ist bekannt und wird in sehr guten Ausbeuten z. B. aus Tetrachlorkohlenstoff, iso-Buten und Vinylchlorid erhalten.

Zu dem durch die neue Verbindung 5,5-Dichlor-3,3-dimethyl-pentansäure (I) eröffneten neuen Weg zur Herstellung von 4,4-Dimethyl-3,4-dihydro-α-pyron ist folgendes zu sagen:

4,4-Dimethyl-6-acetoxy-valerolacton der Formel III wird durch Umsetzung von 3,3-Dimethyl-5-oxo-pentansäure (II) mit Acetanhydrid hergestellt. Die Umsetzung erfolgt in der Flüssigphase unter Erwärmen auf ca. 80° - 140°C, vorzugsweise 100° - 140°C.

Vorzugsweise werden zur schnelleren Reaktionsführung Katalysatoren wie z. B. $ZnCl_2$, $FeCl_3$ oder $BF_3$ zum Reaktionsgemisch hinzugegeben. Es werden mindestens äquivalente Mengen Acetanhydrid eingesetzt, ein Überschuß schadet nicht. Die Verbindung der Formel (II) ist bekannt aus US-A-4 235 780 (siehe dort Spalte 7, Verbindung Id).

Das Reaktionsgemisch kann zur Isolierung und Reindarstellung von 4,4-Dimethyl-6-acetoxy-valerolacton III durch fraktionierte Destillation aufgearbeitet werden. Das Reaktionsgemisch kann jedoch auch ohne Aufarbeitung zu 4,4-Dimethyl-3,4-dihydro-α-pyron der Formel IV weiterverarbeitet werden.

Die Herstellung von 4,4-Dimethyl-3,4-dihydro-α-pyron der Formel IV aus 4,4-Dimethyl-6-acetoxy-valerolacton der Formel III durch Abspaltung von Essigsäure erfolgt bei erhöhten Temperaturen in der Gasphase. Beispielsweise wird die Verbindung der Formel III in flüssiger Form in ein geheiztes Rohr eindosiert, wobei Verdampfung und Reaktion erfolgt. Die Reaktionsgase werden anschließend gekühlt und kondensiert. Die Pyrolyse wird bei Normaldruck oder vermindertem Druck ausgeführt. Das Pyrolyserohr kann mit oder ohne Füllmaterialien eingesetzt werden. Das Füllmaterial hat im wesentlichen den Zweck, als Wärmeüberträger zu dienen. Dafür kommen beispielsweise Füllkörper aus Quarz, Glas, Porzellan, Aluminiumoxid, Ton oder Eisen in Frage. Die zweckmäßig angewandten Pyrolysetemperaturen liegen zwischen 300° und 500°C, vorzugsweise zwischen 350° und 450°C.

In einer vorteilhaften Ausführungsform des Verfahrens wird die Herstellung und Spaltung des 4,4-Dimethyl-6-acetoxy-valerolactons ausgehend von 3,3-dimethyl-5-oxo-pentansäure der Formel II in einer Verfahrensstufe zusammengefaßt.

In diesem Falle wird 3,3-Dimethyl-5-oxo-pentansäure mit Acetanhydrid gegebenenfalls unter Zusatz der genannten Katalysatoren in der Gasphase bei Temperaturen zwischen 300° und 500°C umgesetzt.

Die nach der Gasphasenreaktion erhaltenen Stoffgemische werden durch fraktionierte Vakuumdestillation aufgetrennt.

3,3-Dimethyl-5-oxo-pentansäure wird erhalten, indem man 5,5-Dichlor-3,3-dimethylpentansäure der Formel I mit Basen umsetzt.

Als Basen dienen z. B. Alkali- oder Erdalkalihydroxide, tertiäre Amine, Alkali- oder Erdalkalicarbonate.

Als Lösungsmittel dient bevorzugt Wasser. Es kann aber auch in wasserhaltigen mit Wasser nicht mischbaren Lösungsmitteln wie aliphatischen oder aromatischen Kohlewasserstoffen oder Ethern wie Tetrahydrofuran gearbeitet werden. Als Katalysatoren können übliche Phasentransferkatalysatoren wie Tetralkylammoniumhydroxide zugesetzt werden. Man arbeitet bei Temperaturen von 80 - 120°C, bevorzugt über 90°C.

Man arbeitet bei Normaldruck oder leichtem Überdruck. Die Base wird in äquimolarer Menge, gegebenenfalls einem Überschuß von bis zu 10 %, eingesetzt.

5,5-Dichlor-3,3-dimethylpentansäure braucht für die Reaktionssequenz (I) → (II) → (III) → (IV) nicht rein eingesetzt werden, man kann sie auch in Form des bei seiner Bildung entstehenden Reaktionsgemisches einsetzen.

**Herstellungsbeispiel**

5,5-Dichlor-3,3-dimethyl-pentansäure (I)

500 g 1,1,5,5-Tetrachlor-3,3-dimethyl-penten werden innerhalb 1 Stunde unter Rühren so zu 2400 ml Schwefelsäure zudosiert, daß die Reaktionsmischung nicht über 45°C steigt. Das Reaktionsgemisch wird weitere 24 Stunden bei 40° - 45°C gehalten und dann auf 3 kg Eis gegeben. Die Mischung wird mehrfach mit Dichlormethan extrahiert. Nach dem Abdestillieren des Lösungsmittels erhält man 420 g rohe 5,5-Dichlor-3,3-dimethyl-pentansäure, nach der Destillation 380 g reine 5,5-Dichlor-3,3-dimethyl-pentansäure, Kp. $_{0,2}$ 103 - 106°C.

**Patentansprüche**

1. 5,5-Dichlor-3,3-dimethylpentansäure der Formel I

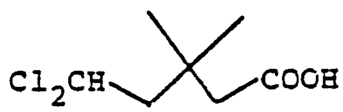

(I)

2. Verfahren zur Herstellung von 5 5-Dichlor-3,3-di-methylpentansäure der Formel (I) gemäß Anspruch 1, dadurch gekennzeichnet, daß man 1,1,5,5-Tetrachlor-3,3-dimethyl-penten der Formel V

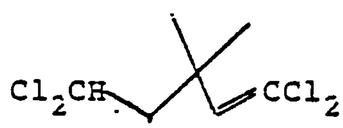

(V)

sauer hydrolysiert.

**Claims**

1. 5,5-Dichloro-3,3-dimethylpentanoic acid of the formula I

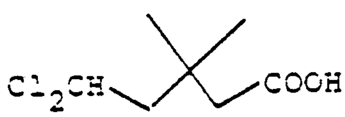

(I)

2. Process for the preparation of 5,5-dichloro-3,3-dimethylpentanoic acid of the formula (I) according to Claim 1, characterized in that 1,1,5,5-tetrachloro-3,3-dimethyl-pentane of the formula V

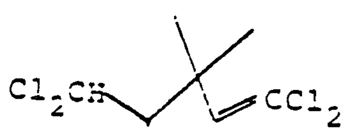

(V)

is subjected to acid hydrolysis.

**Revendications**

1. L'acide 5,5-dichloro-3,3-diméthylpentanoïque de formule I

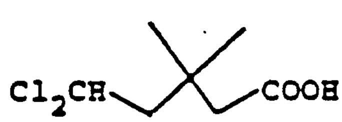

(I)

2. Procédé de préparation de l'acide 5,5-dichloro-3,3-diméthylpentanoïque de formule I selon la revendication 1, caractérisé en ce que l'on soumet le 1,1,5,5-tétrachloro-3,3-diméthylpentène de formule V

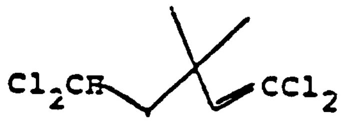 (V)

à hydrolyse acide.